# EUROPEAN PATENT APPLICATION

(11) **EP 1 393 702 A1**
(43) Date of publication of application: **03.03.2004**
(21) Application number: 02019216.7
(22) Date of filing: 27.08.2002
(51) Int. Cl.: A61F 13/42

(54) **Diaper having an electronic wetness indicator system and its manufacturing method**

(71) Applicant: Huang, Chien-Tung, Hua-Lien County (TW); Chuan, Lin Wen, Hsin Chuang City, Taipei County (TW)
(72) Inventor: Huang, Chien-Tung, Hua-Lien County (TW); Chuan, Lin Wen, Hsin Chuang City, Taipei County (TW)
(74) Representative: Onn, Thorsten

(57) **Abstract**

An electronic diaper and its manufacturing method includes a diaper body and sensing wires positioned on the diaper body, and an electronic device to connect with the sensing wires so as to get signal from the sensing wires about the condition of the diaper body. If the sensing wires are connected electrically by urine absorbed by the diaper body, then the electronic device gets signal from the sensing wires to operate to light up an LED or let a speaker sound out so as to give out an alarm that the diaper body has wetted to be replaced with a new one. The electronic device is separatively combined with the diaper body so as to be used repeatedly in spite of the diaper body having to be discarded when wetted by urine.

## Description

### BACKGROUND OF THE INVENTION

At present, some of paper diapers available in the market have a water sensing ink painted on an outer water-proof layer for producing color change according to wetting condition of the diaper by urine absorbed in the diaper so as to let a nurse to know it is time to replace a wet diaper with a new dry one

However, the water sensing ink may produce color change according to the condition of a diaper, but the ink strip is quite narrow, not evident in its color changed, and a nurse has to draw down a little the trouser of a user before the nurse can detect the ink strip whether its color has changed or not. Therefore, no matter the user is an adult or a child, the user may feel uncomfortable if the diaper is not changed in time when it becomes wet with urine. Or if light is not so bright during the night, a nurse may not possible to discern whether the ink has changed color or not owing to impossibility to detect clearly color change of the ink.

Moreover, if a nurse is careless, or lazy, the nurse may not pay attention to the change of the ink strip for replacing a diaper in time, but there is no evidence to prove the nurse purposely do it,

Another point is that the water sensing ink may change its color if there is much moisture in air, and it may cause a nurse to make incorrect judge to replace a diaper even if it is not really wet with urine.

### SUMMARY OF THE INVENTION

A first purpose of the invention is to offer an electronic diaper to give out a warning sound or a lamp lit up in case of a diaper becoming wet with urine.

A second purpose of the invention is to offer an electronic diaper possible to be manufactured by an automatic mode, with sensing wires automatically fixed on a diaper, so as to lower its cost.

The invention has the following features.
1. It has sensing wires adhered on an inside surface of a diaper body, and the sensing wires have their ends extending to an front upper end or a rear upper end of a diaper body, and a plug is connected to each end so as to insert in a socket provided in an electronic device.
2.The manufacturing method in the invention utilizes a long material band, and a plurality of sensing wires are equidistantly adhered on the material band or in an inner surface of a lower dry layer automatically.
3.It includes an electronic device possible to be used repeatedly, lowering the expenditure of a user, and not increasing cost of a diaper body itself.

### BRIEF DESCRIPTION OF DRAWINGS

This invention will be better understood by referring to the accompanying drawings, wherein:
Figure 1 is a cross-sectional view of a first embodiment of sensing wires disposed in a diaper body in the present invention;
Figure 2 is a cross-sectional view of a second embodiment of sensing wires disposed in a diaper body in the present invention;

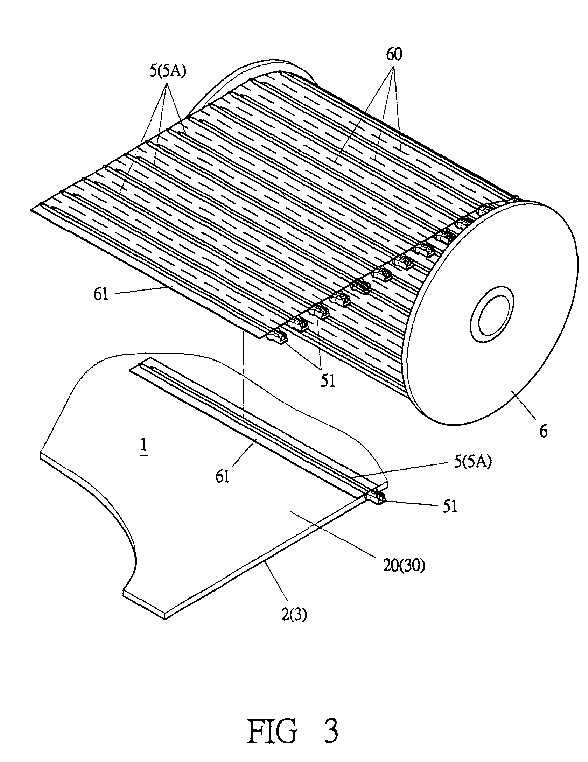

Figure 3 is a perspective view of an electronic diaper being made in the present invention;

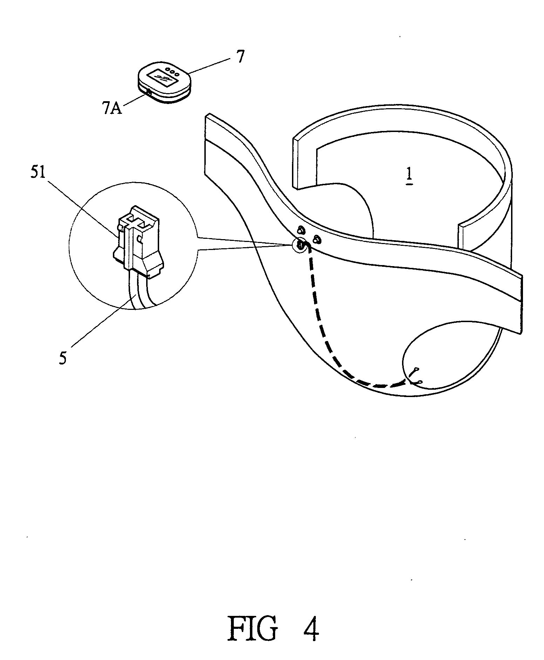

Figure 4 is a perspective and a partial magnified view of a first embodiment of an electronic diaper in the present invention;

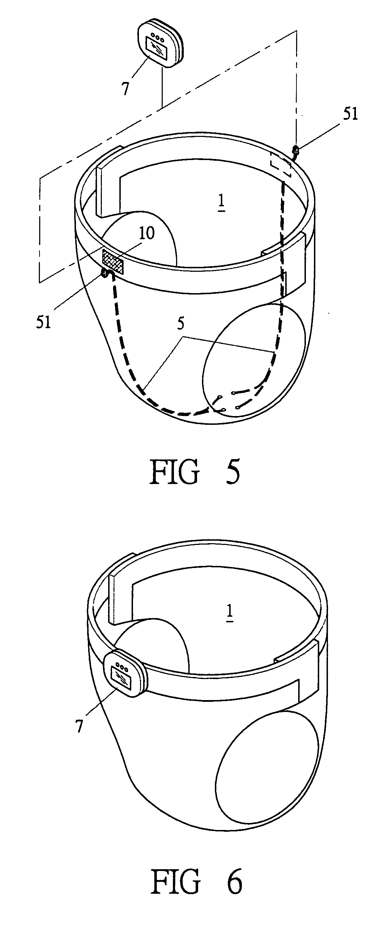

Figure 5 is an exploded perspective view of a second embodiment of an electronic diaper in the present invention;
Figure 6 is a perspective view of the second embodiment of an electronic diaper in the present invention;
Figure 7 is a diagram of a first control circuit for the first embodiment of an electronic diaper in the present invention;
Figure 8 is a diagram of a second control circuit for the second embodiment of an electronic diaper in the present invention; and,
Figure 9 is a flow chart of operation of software of an IC in the present invention.

### DETAILED DESCRIPTION OF THE PREFERRED EMBODIMENT

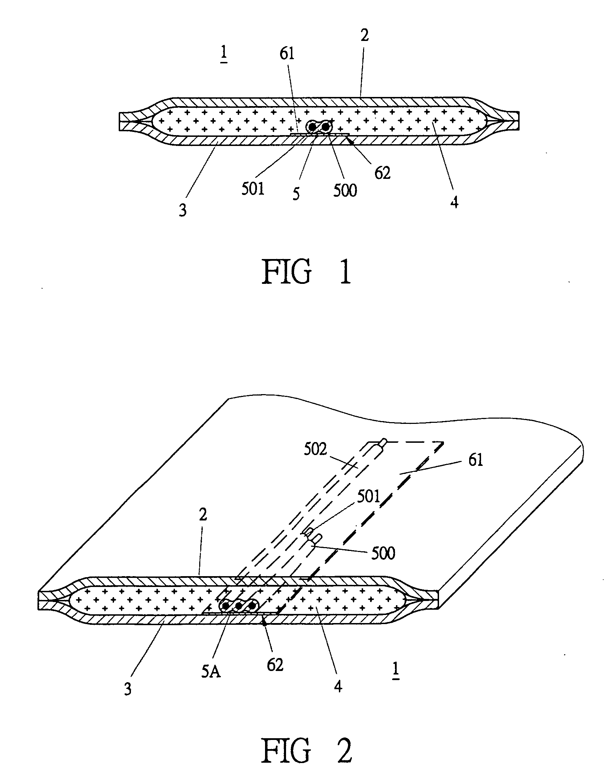

A first preferred embodiment of an electronic diaper in the present invention, as shown in Fig. 1 includes a diaper body 1, a plurality of sensing wires 5, an electronic device 7 and a combining member 10 as main components combined together.

The diaper body 1 consists of an upper dry layer 2, a lower dry layer 3, and an absorbing layer 4 disposed between the upper and the lower dry layer 2 and 3. The diaper body 1 can be made of paper, not woven cloth, plastic, common cloth or wood.

The sensing wires 5 are adhered on an inner surface of either the upper dry layer 2 or the lower dry layer 3 vertically to let one ends of them reaching a front upper end or a rear upper end of the diaper body 1. The sensing wires 5 consist of two leading wires 500 and 501 as shown in Fig. 1.

A plug 51 is connected with one end of each leading wires 500 and 501 so as to insert in a socket provided on an electronic device 7, for convenience of separating the electronic device 7 when the diaper body 1 is to be discarded because of becoming wet with urine, for using the electronic device 7 repeatedly without need of discarding as the diaper body 1.

The combining member 10 may be Velcro bands, separately provided on an outer surface of the electronic device 7 and on an upper end of the diaper body 1 in order to combining or separating easily the electronic device 7 from the diaper body 1 in use.

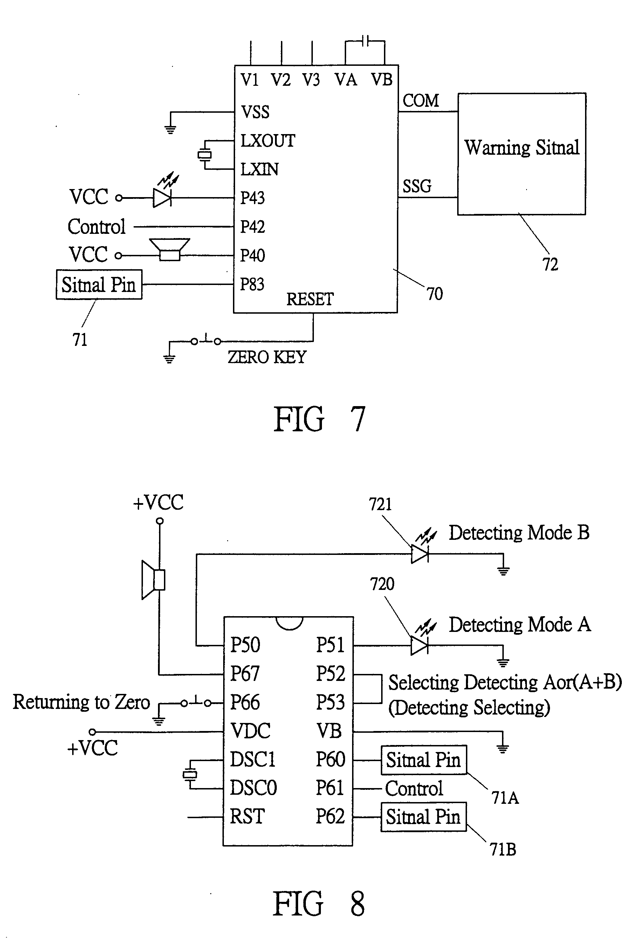

The electronic device 7 contains a control circuit 70 shown in Fig. 7, which is formed in an IC 70 with a drive program burned therein. The IC 70 has a signal pin 71 to connect with the sensing wires 5 so as to transmit signal to the IC 70. Then the IC 70 may operates and sends out an output signal to warning signal 72, Ex. an LED to light up or a speaker to give out sound; and/or an LCD for displaying out the time of the diaper body gotten wet. Then a nurse may see the LED lit up or hear the sound from the speaker and/or see the displayed time on the LCD to decide whether or not to replace the diaper body 1 with a new one. Therefore, the wetted diaper body 1 may be replaced with a new one by the nurse in due time, let a user feel comfortable.

A second embodiment of an electronic diaper is shown in Fig. 2, almost has the same structure as the first embodiment, but the difference is the sensing wires 5a and the electronic device 7.

The sensing wire 5A in the second embodiment has three leading wires 500. 501 and 502, and the two leading wires 500 and 501 are comparatively short and the wire 501 is a common grounding one, but the leading wire 502 is comparatively long. So when the two short leading wires 500 and 501 are connected electrically by urine absorbed by the diaper body 1, the IC 70 may be triggered by signal pin 71A to operate to light up an LED 720 (detecting mode A) of green color, indicating the volume of urine absorbed in the diaper body 1 is small, needless to replace the diaper body 1 yet. But when the short leading wire 501 and the short leading wire 502 are connected electrically by urine by a large volume of urine absorbed in the diaper body 1, the IC 70 may be triggered by signal pin 71B to operate to light up an LED 721 (detecting mode B) of red light. Then it indicates the diaper body 1 is wet enough to be replaced with a new one. Then a nurse may take necessary action at seeing the lit up LED 721 or the sound given out by a speaker without fail.

Further, the electronic device 7 may adopt a continual detecting mode or an intermittent detecting mode to the signal of the sensing wires 5 or 5A. If the continual mode is employed, the IC 70 supplies the sensing wires with micro continual electricity, and if the intermittent mode is employed, the IC 70 supplies the sensing wires with intermittent micro electricity. The intermittent time may be a few seconds or a few minutes, so long as the condition of the diaper body can be detected in a proper time.

A second embodiment of the electronic device 7 to be used in the second embodiment of the electronic diaper is shown in Fig. 8, having two detecting modes A and B, for separately detecting a small volume and a large volume of urine absorbed by the diaper body 1 as described above.

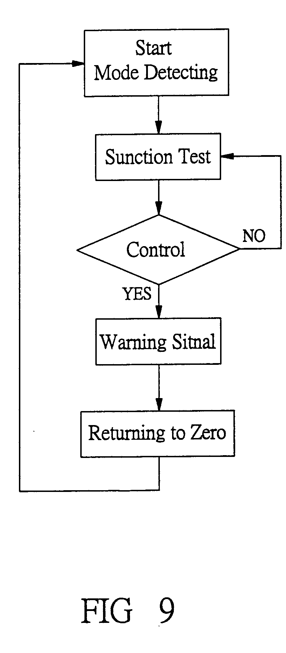

Further, the operational mode of the IC 70 is shown in Fig. 9, using a start mode of detection to test the condition of the system and the function at turning on electricity. The sensing wires 5 or 5A receives continual or intermittent supply of electricity from the electricity device 7 to detect the condition of the diaper body 1. If the signal is "yes", it represents that the diaper body 1 has already wetted enough, and then the control circuit at once turns off electricity to the sensing wires 5 or 5A. But the control circuit 7 still continues to give out signal until a user turns the signal to zero to let the control circuit 70 enter standby condition.

A manifesting method for an electronic diaper includes several steps. A first step is to prepare a long continual band-shaped material for a plurality of diaper bodies, made of paper, no woven cloth, plastic, common cloth, or wood. A second step is to adhere a plurality of sensing wires spaced apart equidistantly on an inner surface of said long band-shaped material by means of glue or high frequency welding and forming a cut line between every two neighboring sensing wires. A third step is to connect a plug with one end of each sensing wire. A fourth step is to wind the long continual band-shaped material finished the third step into a roll. A fifth step is to place the roll of the material on an automatic machine to pull out the material and cut it into a plurality of one piece of the size of a diaper body. A sixth step is to form each piece of diaper body material into a diaper body shape. A seventh step of adhering a combining member on an upper end of each diaper body. And an eighth step is to prepare an electronic device with a socket and a combining member.

While the preferred embodiments of the invention have been described above, it will be recognized and understood that various modifications may be made therein and the appended claims are intended to cover all such modifications that may fall within the spirit and scope of the invention.

## Claims

1. A manufacturing method for an electronic diaper comprising:
a first step of preparing a long continual band-shaped material for a plurality of diaper bodies made of paper, not woven cloth, common cloth, plastic or wood;
a second step of adhering sidewise a plurality of sensing wires spaced apart equidistantly on said long band-shaped material by means of glue or high frequency welding, and forming a cutting line between every neighboring two of said sensing wires;
a third step of connecting a plug with an end of each said sensing wire;
a fourth step of winding said long continual band-shaped material into a roll;
a fifth step of placing said roll of material on an automatic machine to pull out said long continual band-shaped material and cutting into a plurality of one piece of the size of a diaper body;
a sixth step of forming each said piece of material into a diaper body shape;
a seventh step of adhering a combining member on an upper end of each diaper body coming out of the fifth step.

2. The manufacturing method for an electronic diaper as claimed in Claim 1, further comprising an eighth step of preparing an electronic device, said electronic device comprising a control circuit formed into an IC with a drive program burned therein, said IC having a signal pin to connect with said sensing wire.

3. An electronic diaper comprising:
a diaper body consisting of an upper dry layer, a lower dry layer, and an absorbing layer positioned between said upper and said lower layer;
a plurality of sensing wires adhered on an inner surface of said upper dry layer or of said lower dry layer, said sensing wires having respectively one end extending to a front upper end or a rear upper end or a side upper end or to both a front and a rear upper end;
a plug connected with the upper end of each said sensing wire for connecting with a socket of an electronic device.

4. The electronic diaper as claimed in Claim 3, wherein said sensing wires consist of two leading wires.

5. The electronic diaper as claimed in Claim 4, wherein said sensing wires consist of three leading wires.

6. An electronic diaper comprising:
a diaper body having at least an upper dry layer, a lower dry layer, and an absorbing layer positioned between said upper and said lower dry layer;
a plurality of sensing wires adhered on an inner surface of said upper or said lower dry layer;
a plug connected with one end of each said sensing wires so as to connect with a socket of an electronic device; and,
said electronic device having a control circuit formed into an IC, said IC having a drive program burned therein and a signal pin to connect with said sensing wires for transmitting signal to said IC, said IC operating on receiving signal from said sensing wires to send out an output signal to let an LED to light up or a speaker to sound out; and/or an LCD to display the time when said diaper body gets wet enough to be replaced with a new one.

7. The electronic diaper as claimed in Claim 5, wherein said control circuit has two detecting modes in conjunction with said sensing wires consisting of three leading wires, two of said leading wires being comparatively short, one of said two short wires being a grounding one, the third leading wire being comparatively long, said two short wires connected electrically by urine in case the urine absorbed by the diaper body is rather little, in that case said control circuit sending out an alarming signal, said control circuit sending out another signal indicating the diaper body has to be replaced when said third long leading wire and the grounding wire are connected electrically by much urine absorbed by the diaper body.

8. The electronic diaper as claimed in Claim 5, wherein said electronic device has a continual mode of detection against signals by supplying continually micro electricity to said sensing wires for transmitting the condition of the diaper body, or a intermittent mode of detection against signals by supplying intermittently micro electricity to said sensing wires, the electronic device at once cutting off electricity to said sensing wires but continuing to give out signal until the wetted diaper body is replaced when it gets signal of said long sensing wire and said grounding wire connected electrically.
